# EUROPEAN PATENT SPECIFICATION

(11) **EP 4 480 383 B1**
(45) Date of publication and mention of the grant of the patent: **15.07.2026**
(21) Application number: 24174704.7
(22) Date of filing: 08.05.2024
(51) Int. Cl.: A61B 1/00, A61B 1/267, A61B 1/05, A61B 1/06

(54) **DISPOSABLE BLADE LARYNGOSCOPE AND MANUFACTURING METHOD THEREOF**
LARYNGOSKOP MIT EINWEGKLINGE UND HERSTELLUNGSVERFAHREN DAFÜR
LARYNGOSCOPE À LAME JETABLE ET SON PROCÉDÉ DE FABRICATION

(30) Priority: 19.06.2023 CN 202310729658
(43) Date of publication of application: 25.12.2024
(73) Proprietor: Zhuhai Weishi Medical Technology Co., Ltd., Zhuhai Guangdong 519000 (CN)
(72) Inventor: XIONG, Qibiao, Zhuhai, 519000 (CN); CHEN, Jiacheng, Zhuhai, 519000 (CN); QIU, Lianhao, Zhuhai, 519000 (CN); ZHANG, Kunpeng, Zhuhai, 519000 (CN); LIU, Lang, Zhuhai, 519000 (CN); XIE, Ruiliang, Zhuhai, 519000 (CN)
(74) Representative: Metida

(56) References cited:
- US-A1- 2013 060 090
- US-A1- 2013 197 312
- US-A1- 2014 202 459
- US-A1- 2014 371 536
- US-A1- 2022 000 354

## Description

### TECHNICAL FIELD

The present invention relates to the technical field of laryngoscopes, and in particular to a disposable blade laryngoscope as defined in claim 1 and a processing method for obtaining such a laryngoscope as defined in claim 6.

### BACKGROUND

A video laryngoscope is a new choice for difficult clinical tracheal intubation in recent years, on one hand, it solves the limitation of visual field of human eyes, on the other hand, its laryngoscope lens conforms to the anatomical angle of human throat, thereby being beneficial to exposure of glottis and reducing the difficulty of tracheal intubation. The video laryngoscope changes the tracheal intubation mode of a traditional laryngoscope, and shifts the sight line from the outside of the mouth to the front end of the laryngoscope, thus becoming a high-efficiency and convenient tool for solving difficult clinical intubation at present, which obtains more and more extensive clinical application.

The video laryngoscope collects images of epiglottis and glottis anatomical structures through an image sensor at a handle, and transmits the images to a display screen through a video transmission line, whereby a doctor observes the whole process of tracheal intubation, and assists in intubation through the handle. The video laryngoscope generally includes a laryngoscope lens, an optical lens, a display and the like, and the laryngoscope lens contacts the oral mucosa of a patient and needs to meet related biocompatibility test requirements, at least including cytotoxicity, oral mucosa stimulation and sensitization reaction.

A common structure of an existing laryngoscope lens on the market at present is that a shell adopts the fixing modes of screw locking, glue bonding, buckle locking and the like, for example, a disposable visual laryngoscope disclosed by Chinese Patent Publication No. CN218279605U, in which two shells are connected by a buckling mode, while the problems of complicated assembly, long consumed time, poor uniformity, high assembly cost, and low volume production exit. Meanwhile, this kind of laryngoscope adopts a plurality of materials, which is not favorable for control of biocompatibility. In addition, due to buckling installation, gaps are inevitably formed between the two shells, the gaps easily cause the body fluid to enter the inside of the laryngoscope to damage visual components, therefore, the laryngoscope needs to be designed with a sealing structure or a unique buckling mechanism to ensure the sealing performance of the shells, and meanwhile, the gaps on the shells easily hide dirt, which is not beneficial to cleaning and protection.

US 2014/202459 A1 discloses a disposable blade laryngoscope with a blade that comprises a first shell and a second shell.

US 2013/060090 A1 discloses a coating shell for a laryngoscope.

### SUMMARY

In order to overcome one of the defects of the relevant art, an objective of the present disclosure is to provide a disposable blade laryngoscope and a processing method thereof, in which the disposable blade laryngoscope is simple in structure and good in sealing performance, and the processing method is convenient in processing, which are beneficial for improving the sealing performance of the laryngoscope.

In order to solve the above problems, the technical solution adopted by the present invention is as follows.

A disposable blade laryngoscope includes a blade, a coating shell and an optical lens assembly, and the blade includes a first shell and a second shell formed by a primary injection molding process which are buckled to form a mounting channel; one end of the mounting channel has a mounting clamping hole, and the other end forms a mounting fixed seat; the coating shell , which is formed by a secondary injection molding process, coats the outside of the blade, and the end, close to the mounting clamping hole, of the coating shell extends outwards to form a tongue depressor; the end, close to the mounting fixed seat, of the coating shell forms a hollow handle integrally formed with the tongue depressor, and the coating shell is provided with a visible hole communicating with the mounting clamping hole; and the optical lens assembly is installed in the mounting channel, the input end of the optical lens assembly is clamped in the mounting clamping hole and penetrates through the coating shell, and the output end of the optical lens assembly is installed on the mounting fixed seat and placed in the handle.

In some possible implementation modes, the optical lens assembly includes a light source module, a camera module and an interface module, the light source module and the camera module are installed in the mounting clamping hole, the light source module and the camera module are connected with the interface module through data lines, the interface module is installed in the mounting fixed seat, and one end of the interface module extends outwards, is placed in the handle and forms a quick insert joint.

In some possible implementation modes, clamping grooves are formed on the inner sides of the ends, close to the mounting clamping hole, of the first shell and the second shell, a transparent window plate is clamped in the clamping groove, and the transparent window plate blocks the mounting clamping hole.

In some possible implementation modes, a plurality of positioning bars are disposed in the first shell and the second shell, and the positioning bars on the first shell and the second shell may be matched with each other to clamp the outer wall of the camera module.

In some possible implementation modes, the first shell and the second shell are connected by ultrasonic welding.

A processing method for obtaining a disposable blade laryngoscope according to the invention includes the following steps:
S100: a first shell and a second shell are obtained by primary injection molding, the first shell and the second shell are buckled to form a blade, the blade has a mounting channel inside, and a mounting clamping hole and a mounting fixed seat are disposed at two ends of the mounting channel;
S200: the optical assembly, comprising a light source module, a camera module and an interface module is installed in the mounting channel, the light source module and the camera module are installed in the mounting clamping hole, the interface module is installed on the mounting fixed seat, with the outputting end extending out of the mounting fixed seat, and the light source module and the camera module are connected with the interface module through data lines;
S300: gaps formed after the first shell and the second shell are buckled are sealed by welding through an ultrasonic welding device, thereby obtaining a primary product;
S400: a protection structure is plugged in the mounting clamping hole of the primary product, a protection structure sleeves the part, extending out of the mounting fixed seat, of the interface module, and secondary injection molding of the coating shell is carried out outside the primary product to obtain a product formed by secondary injection molding, wherein the hollow handle is integrally formed with the tongue depressor;
S500: the protection structure on the product of the secondary injection molding is detached, and the coating shell is trimmed to obtain the laryngoscope.

In some possible implementation modes, positioning bars are formed at one ends, with the mounting clamping hole, of the first shell and the second shell obtained by primary injection molding in S100 by injection molding; and after the first shell and the second shell are buckled to form the blade, the positioning bars on the first shell and the second shell form part of the structure of the mounting clamping hole.

In some possible implementation modes, in S400, the coating shell of secondary injection molding outside the primary product has a tongue depressor and a hollow handle, the protection structure coating the part, extending out of the mounting fixed seat, of the interface module forms a filler in the injection molding process of the handle, and the production structure plugged in the mounting clamping hole forms a filler of a visible hole in the coating shell.

In some possible implementation modes, in S200, the light source module, the camera module and the interface module are coated with thermal insulation films and installed in the mounting channel together with the thermal insulation films.

In some possible implementation modes, in S200, when the light source module and the camera module are installed in the mounting clamping hole, a transparent window plate needs to be installed at the outward end of the mounting clamping hole.

Compared with the relevant art, the present disclosure has the following beneficial effects.

For the disposable blade laryngoscope of the present disclosure, the first shell and the second shell are designed on the basis of the original laryngoscope, which is beneficial for installing the optical lens assembly, and meanwhile, the blade may serve as a protection shell of the optical lens assembly, whereby the optical lens assembly may be supported in a use process to be prevented from being pressed to deform by the epiglottis of the human body in the use process. In addition, one layer of coating shell is formed on the blade by secondary injection molding, whereby the first shell and the second shell may be effectively sealed and fixed, the installation process may be reduced, and the assembly efficiency may be improved; and meanwhile, according to the invention, the tongue depressor and the handle are integrally formed, compared with the traditional forming mode of forming the tongue depressor and the handle on the blade, the manufacturing difficulty of a mold is greatly reduced, which is conducive to demolding of the mold and improving the efficiency of processing. For the processing method of the disposable blade laryngoscope, the operation is simple, the assembly is convenient, and the sealing performance of a finished product is good.

### BRIEF DESCRIPTION OF THE DRAWINGS

In order to describe the technical solutions in the embodiments of the present disclosure more clearly, the drawings required to be used in description of the embodiments will be simply introduced below, obviously, the drawings described below are only some embodiments of the present disclosure, and other drawings can further be obtained by those of ordinary skill in the art according to the drawings without creative work.
Fig. 1 is a schematic structure diagram I of an embodiment of the present disclosure.
Fig. 2 is a schematic structure diagram II of an embodiment of the present disclosure.
Fig. 3 is a schematic internal structure diagram of an embodiment of the present disclosure.
Fig. 4 is a schematic structure diagram with a coating shell removed of an embodiment of the present disclosure.
Fig. 5 is a schematic diagram of an exploded view with a coating shell removed of an embodiment of the present disclosure.

### Description of Reference Numerals:

Blade 10; First shell 11; Second shell 12; Mounting channel 13; Mounting clamping hole 14; Mounting fixed seat 15; Clamping groove 16; Transparent window plate 17; Positioning bar 18;
Coating shell 20; Tongue depressor 21; Handle 22; Visible hole 23;
Optical lens assembly 30; Light source module 31; Camera module 32; Interface module 33; Data line 34; and Quick insert joint 35.

### DETAILED DESCRIPTION OF THE EMBODIMENTS

The technical solutions in the embodiments of the present disclosure are clearly and completely described below in conjunction with the accompanying drawings in the embodiments of the present disclosure. Apparently, the embodiments described are only a part rather than all of the embodiments of the present disclosure. In the following, terms "first" and "second" are only adopted for description and should not be understood to indicate or imply relative importance or implicitly indicate the number of indicated technical features. Therefore, a feature defined by "first" and "second" may explicitly or implicitly indicates inclusion of at least one such feature. In the description of the embodiment of the present disclosure, "a plurality of" means two or more, unless otherwise specified.

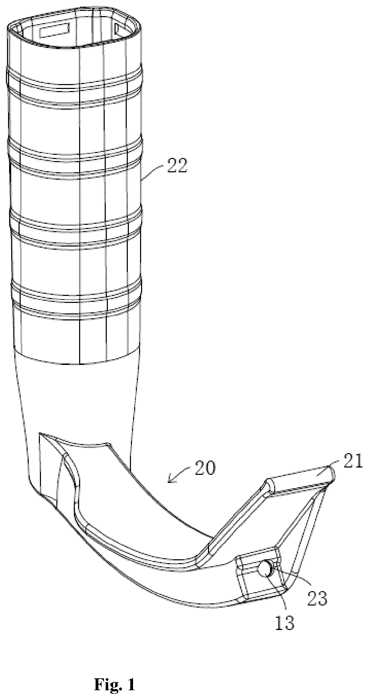

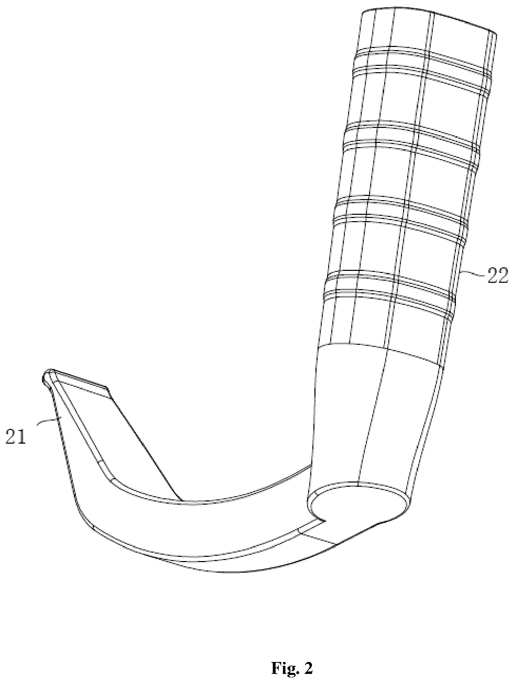

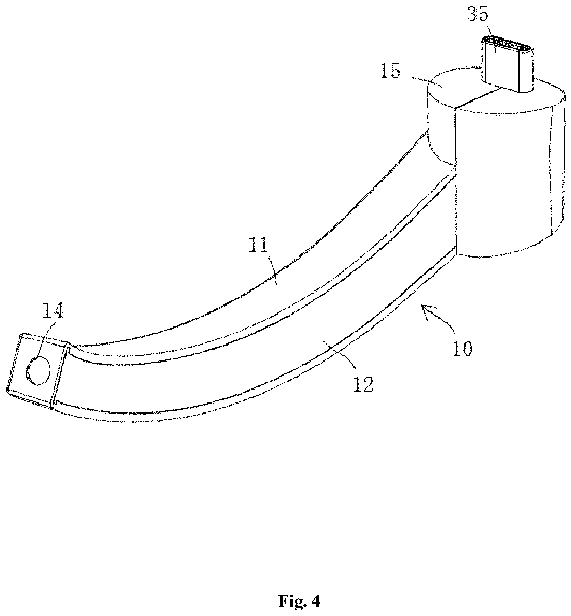

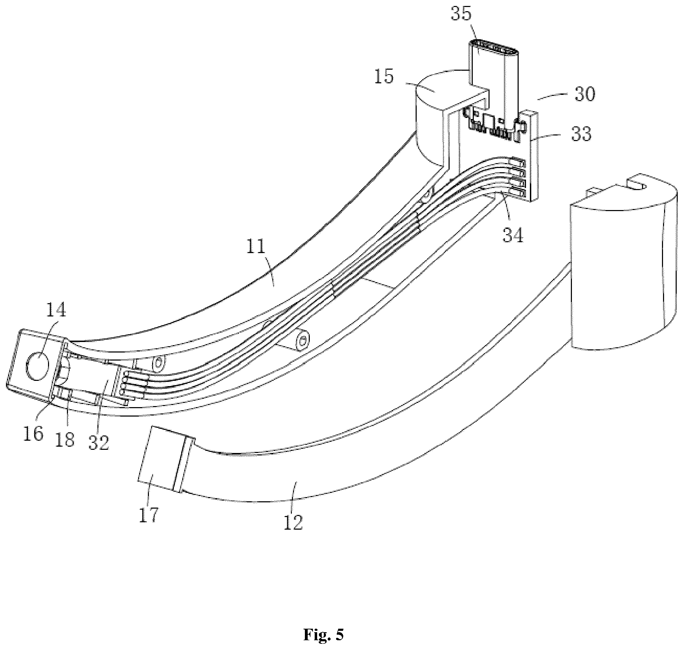

A disposable blade laryngoscope as shown in Figs. 1-5 includes a blade 10, a coating shell 20 and an optical lens assembly 30, the blade 10 includes a first shell 11 and a second shell 12, and the first shell 11 and a second shell 12 are buckled to form a mounting channel 13; one end of the mounting channel 13 has a mounting clamping hole 14, and the other end forms a mounting fixed seat 15; the coating shell 20 coats outside the blade 10, and the end, close to the mounting clamping hole 14, of the coating shell 20 extends outwards to form a tongue depressor 21; the end, close to the mounting fixed seat 15, of the coating shell 20 forms a hollow handle 22, and the coating shell 20 is provided with a visible hole 23 communicating with the mounting clamping hole 14; and the optical lens assembly 30 is installed in the mounting channel 13, the input end of the optical lens assembly 30 is clamped in the mounting clamping hole 14 and penetrates through the coating shell 20, and the output end of the optical lens assembly 30 is installed on the mounting fixed seat 15 and placed in the handle 22.

The first shell 11 and the second shell 12 are obtained by independent processing in a primary forming mold, at least one of the first shell 11 and the second shell 12 has an inner cavity inside to form the mounting channel 13, whereby a mold for forming the first shell 11 or the second shell 12 needs a mold core to form the mounting channel 13, in such a case, if the tongue depressor 21 and the hollow handle 22 are formed in the mold, the processing difficulty of the corresponding mold and the difficulty of demolding of the mold in the later stage may be increased obviously. While the tongue depressor 21 and the hollow handle 22 do not need to be formed on the mold for forming the first shell 11 and the second shell 12 in the present disclosure, therefore, the mold for forming the first shell 11 and the second shell 12 is simpler, and the production cost may be effectively reduced.

For the disposable blade laryngoscope, the first shell 11 and the second shell 12 are designed on the basis of the original laryngoscope, which is beneficial for installing the optical lens assembly 30, and meanwhile, the blade 10 may serve as a protection shell of the optical lens assembly 30, whereby the optical lens assembly 30 may be supported in a use process to be prevented from being pressed to deform by the epiglottis of the human body in the use process. In addition, one layer of coating shell 20 is formed on the blade 10 by secondary injection molding, whereby the first shell 11 and the second shell 12 may be effectively sealed and fixed, the installation process may be reduced, and the assembly efficiency may be improved; and meanwhile, according to the invention, the tongue depressor 21 and the handle 22 are integrally formed, compared with the traditional forming mode of forming the tongue depressor 21 and the handle 22 on the blade 10, the manufacturing difficulty of a mold is greatly reduced, which is conducive to demolding of the mold and improving the efficiency of processing.

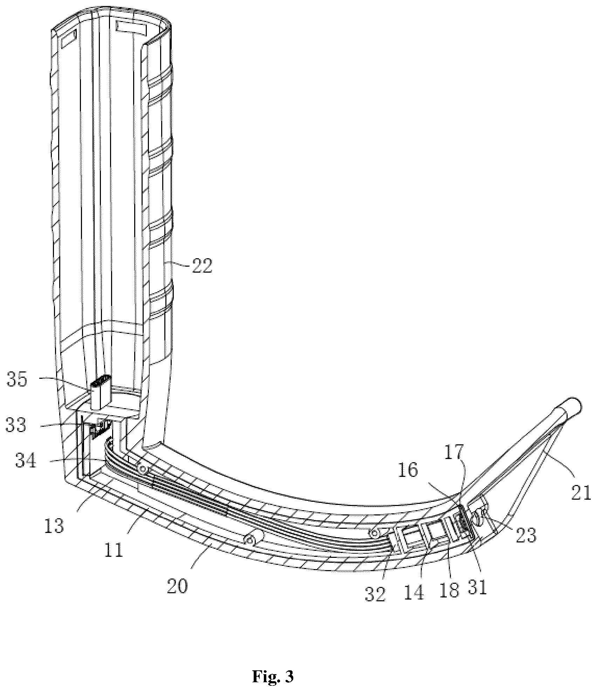

Furthermore, referring to Figs. 3-5, in some possible implementation modes, the optical lens assembly 30 includes a light source module 31, a camera module 32 and an interface module 33, the light source module 31 and the camera module 32 are installed in the mounting clamping hole 14, the light source module 31 and the camera module 32 are connected with the interface module 33 through data lines 34, the interface module 33 is installed in the mounting fixed seat 15, and one end of the interface module 33 extends outwards, is placed in the handle 22 and forms a quick insert joint 35. The camera module 32 and the light source module 31 are ready-made modules, which will not be elaborated in the present disclosure, and reference can be made to a camera module in a laryngoscope blade disclosed by Chinese Patent Publication No. CN203634128U. During installation, the data line 34 and the interface module 33, the light source module 31 and the camera module 32 are respectively connected by welding, a special welding device or welding jig may be adopted for welding, the operation is relatively simple, and during installation, it only needs to clamp the camera module 32 and the interface module 33 into the mounting clamping hole 14 and the mounting fixed seat 15, the entire installation process is relatively simple.

It needs to be noted that in some cases, the light source module 31 may be integrated with the camera module 32 to simplify the structure, of course, in some embodiments, the light source module 31 and the camera module 32 are separate modules, so the light source module 31 needs to be installed separately on the peripheral side of the mounting clamping hole 14. Of course, in some embodiments, for better installation, the light source module 31 may be designed in a ring shape, and then sleeves the front end of the camera module 32 and is clamped in the mounting clamping hole 14 together.

In some possible implementation modes, in order to facilitate protection of the light source module 31 and the camera module 32 and also improve the sealing performance of the blade 10, clamping grooves 16 are formed on the inner sides of the ends, close to the mounting clamping hole 14, of the first shell 11 and the second shell 12, a transparent window plate 17 is clamped in the clamping groove 16, and the transparent window plate 17 blocks the mounting clamping hole 14. The transparent window plate 17 may protect the light source module 31 and the camera module 32 to a certain degree during secondary injection molding of the coating shell 20.

In some possible implementation modes, in order to improve installation precision of the camera module 32, a plurality of positioning bars 18 are disposed in the first shell 11 and the second shell 12, and the positioning bars 18 on the first shell 11 and the second shell 12 may be matched with each other to clamp the outer wall of the camera module 32. In fact, the positioning bars 18 form part of the structure of the mounting clamping hole 14.

In some possible implementation modes, in order to avoid loose of the first shell 11 and the second shell 12 during the secondary injection molding, and further improving the strength and tightness of the entire blade 10, the first shell 11 and the second shell 12 are connected by ultrasonic welding.

The present disclosure further provides a processing method of a disposable blade laryngoscope. The processing method is used for processing the disposable blade laryngoscope of the above embodiment, and includes the following steps:
S100: a first shell 11 and a second shell 12 are obtained by primary injection molding, the first shell 11 and the second shell 12 are buckled to form a blade 10, the blade 10 has a mounting channel 13 inside, and a mounting clamping hole 14 and a mounting fixed seat 15 are disposed at two ends of the mounting channel;
S200: a light source module 31, a camera module 32 and an interface module 33 are installed in the mounting channel 13, the light source module 31and the camera module 32 are installed in the mounting clamping hole 14, the interface module 33 is installed on the mounting fixed seat 15, with the outputting end extending out of the mounting fixed seat 15, and the light source module 31 and the camera module 32 are connected with the interface module 33 through data lines 34;
S300: gaps formed after the first shell 11 and the second shell 12 are buckled are sealed by welding through an ultrasonic welding device, thereby obtaining a primary product;
S400: a protection structure is plugged in the mounting clamping hole 14 of the primary product, a protection structure sleeves the part, extending out of the mounting fixed seat 15, of the interface module 33, and secondary injection molding of a coating shell 20 is carried out outside the primary product to obtain a product of second injection molding;
S500: the protection structure on the product of second injection molding is detached, and the coating shell 20 is trimmed to obtain the laryngoscope.

In S100, molds for injection molding of the first shell 11 and the second shell 12 are independent molds, whereby the first shell 11 and the second shell 12 may be independently formed; while the appearance of a mold for secondary injection molding is designed based on the appearance of the laryngoscope. In addition, in the present disclosure, during secondary injection molding, the whole laryngoscope may be fixed in the mold for secondary injection molding by the corresponding protection structures on the mounting clamping hole 14 and the mounting fixed seat 15. For the processing method of the disposable blade laryngoscope, the operation is simple, the assembly is convenient, and the sealing performance of a finished product is good.

In some possible implementation modes, positioning bars 18 are formed at one ends, with the mounting clamping hole 14, of the first shell 11 and the second shell 12 obtained by primary injection molding in S100 by injection molding; and after the first shell 11 and the second shell 12 are buckled to form the blade 10, the positioning bars 18 on the first shell 11 and the second shell 12 form part of the structure of the mounting clamping hole 14. In order to facilitate injection molding, the first shell 11 and the second shell 12 may adopt the same design, the two are buckled to form a complete blade 10, and in such a case, the blade 10 is in symmetric design.

In some possible implementation modes, in S400, the coating shell 20 of secondary injection molding outside the primary product has a tongue depressor 21 and a hollow handle 22, the protection structure coating the part, extending out of the mounting fixed seat 15, of the interface module 33 forms a filler in the injection molding process of the handle 22, and the production structure plugged in the mounting clamping hole 14 forms a filler of a visible hole 23 in the coating shell 20. With adoption of secondary forming of the tongue depressor 21 and the hollow handle 22, the processing difficulty of the forming molds of the first shell 11 and the second shell 12 may be reduced to a certain degree, which is beneficial for demolding of the molds.

In some possible implementation modes, in order to avoid the influence of a high-temperature molten material on the light source module 31, the camera module 32 and the interface module 33 in the blade 10 during the secondary injection molding process, in S200, the light source module 31, the camera module 32 and the interface module 33 are coated with thermal insulation films and installed in the mounting channel 13 together with the thermal insulation films. Of course, in the present disclosure, the first shell 11 and the second shell 12 are also made of a thermal insulation material, meanwhile, the coating shell 20 may be made of silica gel or a PP material, and the melting point of PP is relatively low, whereby the influence of high temperature on the light source module 31, the camera module 32 and the interface module 33 during the production process may be avoided; and at the same time, rapid cooling is required after the secondary injection molding to reduce the influence on the light source module 31, the camera module 32 and the interface module 33.

In some possible implementation modes, S200 further includes that when the light source module 31 and the camera module 32 are installed in the mounting clamping hole 14, a transparent window plate 17 needs to be installed at the outward end of the mounting clamping hole 14, and the protection structure may plug the mounting clamping hole 14 and protect the transparent window plate 17.

The implementation modes described above are only preferred implementation modes of the present disclosure and are not intended to limit the scope of protection of the present invention, which is solely defined by the appended set of claims.

## Claims

1. A disposable blade laryngoscope, comprising:
a blade (10), comprising a first shell (11) and a second shell (12) formed by a primary injection molding process, wherein the first shell (11) and the second shell (12) are buckled to form a mounting channel (13), one end of the mounting channel (13) has a mounting clamping hole (14), and an other end forms a mounting fixed seat (15);
a coating shell (20) formed by a secondary injection molding process, wherein the coating shell (20) coats the outside of the blade (10), an end, close to the mounting clamping hole (14), of the coating shell (20) extends outwards to form a tongue depressor (21), the end, close to the mounting fixed seat (15), of the coating shell (20) forms a hollow handle (22) integrally formed with the tongue depressor (21), and the coating shel (20) is provided with a visible hole (23) communicating with the mounting clamping hole (14), and
an optical lens assembly (30) installed in the mounting channel (13), wherein an input end of the optical lens assembly (30) is clamped in the mounting clamping hole (14) and penetrates through the coating shell (20), and an output end of the optical lens assembly (30) is installed on the mounting fixed seat (15) and placed in the handle (22).

2. The disposable blade laryngoscope according to claim 1, wherein the optical lens assembly (30) comprises a light source module (31), a camera module (32) and an interface module (33), the light source module (31) and the camera module (32) are installed in the mounting clamping hole (14), the light source module (31) and the camera module (32) are connected with the interface module (33) through data lines (34), the interface module (33) is installed in the mounting fixed seat (15), and one end of the interface module (33) extends outwards, is placed in the handle (22) and forms a quick inser joint (35).

3. The disposable blade laryngoscope according to claim 2, wherein clamping grooves (16) are formed on inner sides of the ends, close to the mounting clamping hole (14), of the first shel (11) and the second shell (12), a transparent window plate (17) is clamped in the clamping grooves (16) and the transparent window plate (17) blocks the mounting clamping hole (14).

4. The disposable blade laryngoscope according to claim 2, wherein a plurality of positioning bars (18) are disposed in the first shell (11) and the second shell (12), and the positioning bar (18) on the first shell (11) and and the second shell (12) are arranged to matched with each other to clamp an outer wall of the camera module (32).

5. The disposable blade laryngoscope according to claim 1, wherein the first shell (11) and the second shell (12) are connected by ultrasonic welding.

6. A processing method for obtaining the disposable blade laryngoscope according to claim 1, comprising the following steps:
S100: carrying out primary injection molding to obtain a first shell (11) and a second shell (12), the first shell (11) and the second shell (12) are buckled to form a blade (10), the blade (10) has a mounting channel (13) inside, and a mounting clamping hole (14) and a mounting fixed seat (15) are disposed at two ends of the mounting channel (13);
S200: installing the optical lens assembly (30), comprising a light source module (31), a camera module (32) and an interface module (33) in the mounting channel (13), the light source module (31) and the camera module (32) are installed in the mounting clamping hole (14), the interface module (33) is installed on the mounting fixed seat (15), with the outputting end extending out of the mounting fixed seat (15), and the light source module (31) and the camera module (32) are connected with the interface module (33) through data lines (34);
S300: sealing gaps formed after the first shell (11) and the second shell (12) are buckled by welding through an ultrasonic welding device, thereby obtaining a primary product;
S400: plugging a protection structure in the mounting clamping hole (14) of the primary product, sleeving a protection structure on the part, extending out of the mounting fixed seat (15), of the interface module (33), and carrying out secondary injection molding of the coating shel (20) outside of the primary product to obtain a product formed by secondary injection molding, wherein the hollow handle (22) is integrally formed with the tongue depressor (21);
S500: detaching the protection structure on the product of the secondary injection molding, and trimming the coating shell (20) to obtain the laryngoscope.

7. The processing method according to claim 6, wherein in S100, positioning bars (18) are formed at the ends with the mounting clamping hole (14) of the first shell (11) and the second shell (12) by injection molding, and after the first shell (11) and the second shell (12) are buckled to form the blade (10), the positioning bars (18) on the first shell (11) and the second shell (12) form part of the structure of the mounting clamping hole (14).

8. The processing method according to claim 6, wherein in S400, the coating shell (20) of secondary injection molding outside the primary product has a tongue depressor (21) and a hollow handle (22), the protection structure coating the part, extending out of the mounting fixed seat (15), of the interface module (33) forms a filler in the injection molding process of the handle (22), and the production structure plugged in the mounting clamping hole (14) forms a filler of a visible hole (23) in the coating shell (20).

9. The processing method according to claim 6, wherein in S200, the light source module (31), the camera module (32) and the interface module (33) are coated with thermal insulation films and installed in the mounting channel (13) together with the thermal insulation films.

10. The processing method according to claim 6, wherein in S200, when the light source module (31) and the camera module (32) are installed in the mounting clamping hole (14), a transparent window plate (17) is installed at the outward end of the mounting clamping hole (14).

## Patentansprüche

1. Einweg-Spatel-Laryngoskop, umfassend:
einen Spatel (10), der eine erste Schale (11) und eine zweite Schale (12) umfasst, die durch ein primäres Spritzgießen gebildet sind, wobei die erste Schale (11) und die zweite Schale (12) miteinander verrastet sind, um einen Montagekanal (13) zu bilden, wobei ein Ende des Montagekanals (13) ein Montageklemmloch (14) aufweist und ein anderes Ende einen Montagebefestigungssitz (15) bildet;
eine durch ein sekundäres Spritzgießen gebildete Ummantelungsschale (20), wobei die Ummantelungsschale (20) die Außenseite des Spatels (10) ummantelt, ein dem Montageklemmloch (14) nahes Ende der Ummantelungsschale (20) sich nach außen erstreckt, um einen Zungenniederdrücker (21) zu bilden, das dem Montagebefestigungssitz (15) nahe Ende der Ummantelungsschale (20) einen hohlen Griff (22) bildet, der einstückig mit dem Zungenniederdrücker (21) ausgebildet ist, und die Ummantelungsschale (20) mit einem Sichtloch (23) versehen ist, das mit dem Montageklemmloch (14) in Verbindung steht; und
eine optische Linsenanordnung (30), die in dem Montagekanal (13) installiert ist, wobei ein Eingangsende der optischen Linsenanordnung (30) in dem Montageklemmloch (14) geklemmt ist und die Ummantelungsschale (20) durchdringt und ein Ausgangsende der optischen Linsenanordnung (30) an dem Montagebefestigungssitz (15) installiert und in dem Griff (22) angeordnet ist.

2. Einweg-Spatel-Laryngoskop nach Anspruch 1, wobei die optische Linsenanordnung (30) ein Lichtquellenmodul (31), ein Kameramodul (32) und ein Schnittstellenmodul (33) umfasst, das Lichtquellenmodul (31) und das Kameramodul (32) in dem Montageklemmloch (14) installiert sind, das Lichtquellenmodul (31) und das Kameramodul (32) über Datenleitungen (34) mit dem Schnittstellenmodul (33) verbunden sind, das Schnittstellenmodul (33) in dem Montagebefestigungssitz (15) installiert ist und ein Ende des Schnittstellenmoduls (33) nach außen vorsteht, in dem Griff (22) angeordnet ist und einen Schnellsteckanschluss (35) bildet.

3. Einweg-Spatel-Laryngoskop nach Anspruch 2, wobei an Innenseiten der dem Montageklemmloch (14) nahen Enden der ersten Schale (11) und der zweiten Schale (12) Klemmnuten (16) ausgebildet sind, eine transparente Fensterplatte (17) in den Klemmnuten (16) geklemmt ist und die transparente Fensterplatte (17) das Montageklemmloch (14) blockiert.

4. Einweg-Spatel-Laryngoskop nach Anspruch 2, wobei mehrere Positionierungsstäbe (18) in der ersten Schale (11) und der zweiten Schale (12) angeordnet sind und die Positionierungsstäbe (18) an der ersten Schale (11) und der zweiten Schale (12) so angeordnet sind, dass sie zusammenpassen, um eine Außenwand des Kameramoduls (32) einzuklemmen.

5. Einweg-Spatel-Laryngoskop nach Anspruch 1, wobei die erste Schale (11) und die zweite Schale (12) durch Ultraschallschweißen verbunden sind.

6. Verfahren zur Herstellung des Einweg-Spatel-Laryngoskops nach Anspruch 1, umfassend die folgenden Schritte:
S100: Durchführen eines ersten Spritzgießens, um eine erste Schale (11) und eine zweite Schale (12) zu erhalten, wobei die erste Schale (11) und die zweite Schale (12) miteinander verrastet werden, um einen Spatel (10) zu bilden, der Spatel (10) innen einen Montagekanal (13) aufweist und ein Montageklemmloch (14) sowie ein Montagebefestigungssitz (15) an zwei Enden des Montagekanals (13) angeordnet sind;
S200: Installieren der optischen Linsenanordnung (30), die ein Lichtquellenmodul (31), ein Kameramodul (32) und ein Schnittstellenmodul (33) umfasst, in dem Montagekanal (13), wobei das Lichtquellenmodul (31) und das Kameramodul (32) in dem Montageklemmloch (14) installiert werden, das Schnittstellenmodul (33) an dem Montagebefestigungssitz (15) installiert wird, wobei sich das Ausgangsende des Schnittstellenmoduls aus dem Montagebefestigungssitz (15) heraus erstreckt, und das Lichtquellenmodul (31) und das Kameramodul (32) über Datenleitungen (34) mit dem Schnittstellenmodul (33) verbunden werden;
S300: Abdichten von Spalten, die nach dem Verrasten der ersten Schale (11) und der zweiten Schale (12) gebildet sind, durch Schweißen mittels einer Ultraschallschweißvorrichtung, wodurch ein Primärprodukt erhalten wird;
S400: Einsetzen einer Schutzstruktur in das Montageklemmloch (14) des Primärprodukts, Aufstecken einer Schutzstruktur auf den aus dem Montagebefestigungssitz (15) herausragenden Teil des Schnittstellenmoduls (33) und Durchführen eines sekundären Spritzgießens der Ummantelungsschale (20) außerhalb des Primärprodukts, um ein durch das sekundäre Spritzgießen gebildetes Produkt zu erhalten, wobei der hohle Griff (22) einstückig mit dem Zungenniederdrücker (21) ausgebildet ist;
S500: Entfernen der Schutzstruktur an dem Produkt des sekundären Spritzgießens und Beschneiden der Ummantelungsschale (20), um das Laryngoskop zu erhalten.

7. Verfahren nach Anspruch 6, wobei in S100 Positionierungsstäbe (18) an den mit dem Montageklemmloch (14) versehenen Enden der ersten Schale (11) und der zweiten Schale (12) durch Spritzgießen gebildet werden, und, nachdem die erste Schale (11) und die zweite Schale (12) miteinander verrastet worden sind, um den Spatel (10) zu bilden, die Positionierungsstäbe (18) an der ersten Schale (11) und der zweiten Schale (12) einen Teil der Struktur des Montageklemmlochs (14) bilden.

8. Verfahren nach Anspruch 6, wobei in S400 die Ummantelungsschale (20) des sekundären Spritzgießens außerhalb des Primärprodukts einen Zungenniederdrücker (21) und einen hohlen Griff (22) aufweist, die den aus dem Montagebefestigungssitz (15) herausragenden Teil des Schnittstellenmoduls (33) ummantelnde Schutzstruktur im Spritzgießprozess des Griffs (22) einen Füllkörper bildet und die in das Montageklemmloch (14) eingesetzte Schutzstruktur einen Füllkörper für ein Sichtloch (23) in der Ummantelungsschale (20) bildet.

9. Verfahren nach Anspruch 6, wobei in S200 das Lichtquellenmodul (31), das Kameramodul (32) und das Schnittstellenmodul (33) mit Wärmeisolierfolien ummantelt und zusammen mit den Wärmeisolierfolien in dem Montagekanal (13) installiert werden.

10. Verfahren nach Anspruch 6, wobei in S200, wenn das Lichtquellenmodul (31) und das Kameramodul (32) in dem Montageklemmloch (14) installiert werden, eine transparente Fensterplatte (17) an dem nach außen gewandten Ende des Montageklemmlochs (14) angebracht wird.

## Revendications

1. Laryngoscope à lame jetable, comprenant :
une lame (10), comprenant une première coque (11) et une seconde coque (12) formées par un processus de moulage par injection primaire, dans laquelle la première coque (11) et la seconde coque (12) sont encliquetées pour former un canal de montage (13), une extrémité du canal de montage (13) comporte un trou de serrage de montage (14), et l'autre extrémité forme un siège fixe de montage (15) ;
une coque de revêtement (20) formée par un processus de moulage par injection secondaire, dans laquelle la coque de revêtement (20) recouvre l'extérieur de la lame (10), une extrémité proche du trou de serrage de montage (14) de la coque de revêtement (20) s'étend vers l'extérieur pour former un abaisse-langue (21), l'extrémité proche du siège fixe de montage (15) de la coque de revêtement (20) forme une poignée creuse (22) formée intégralement avec l'abaisse-langue (21), et la coque de revêtement (20) est pourvue d'un trou visible (23) en communication avec le trou de serrage de montage (14), et un assemblage de lentille optique (30) installé dans le canal de montage (13), une extrémité d'entrée de l'assemblage de lentille optique (30) étant serrée dans le trou de serrage de montage (14) et pénétrant à travers la coque de revêtement (20), et une extrémité de sortie de l'assemblage de lentille optique (30) étant installée sur le siège fixe de montage (15) et placée dans la poignée (22).

2. Laryngoscope à lame jetable selon la revendication 1, dans lequel l'assemblage de lentille optique (30) comprend un module de source lumineuse (31), un module de caméra (32) et un module d'interface (33), le module de source lumineuse (31) et le module de caméra (32) sont installés dans le trou de serrage de montage (14), le module de source lumineuse (31) et le module de caméra (32) sont connectés au module d'interface (33) par des lignes de données (34), le module d'interface (33) est installé dans le siège fixe de montage (15), et une extrémité du module d'interface (33) s'étend vers l'extérieur, est placée dans la poignée (22) et forme un joint d'insertion rapide (35).

3. Laryngoscope à lame jetable selon la revendication 2, dans lequel des rainures de serrage (16) sont formées sur des côtés internes des extrémités, proches du trou de serrage de montage (14) de la première coque (11) et de la seconde coque (12), une plaque de fenêtre transparente (17) est serrée dans les rainures de serrage (16) et la plaque de fenêtre transparente (17) bloque le trou de serrage de montage (14).

4. Laryngoscope à lame jetable selon la revendication 2, dans lequel une pluralité de barres de positionnement (18) sont disposées dans la première coque (11) et la seconde coque (12), et la barre de positionnement (18) sur la première coque (11) et la seconde coque (12) sont agencées pour correspondre l'une à l'autre afin de serrer une paroi externe du module de caméra (32).

5. Laryngoscope à lame jetable selon la revendication 1, dans lequel la première coque (11) et la seconde coque (12) sont connectées par soudage ultrasonique.

6. Procédé de traitement pour obtenir le laryngoscope à lame jetable selon la revendication 1, comprenant les étapes suivantes :
S100 : exécuter un moulage par injection primaire pour obtenir une première coque (11) et une seconde coque (12), la première coque (11) et la seconde coque (12) étant encliquetées pour former une lame (10), la lame (10) comportant un canal de montage (13) à l'intérieur, et un trou de serrage de montage (14) et un siège fixe de montage (15) étant disposés à deux extrémités du canal de montage (13) ;
S200 : installer l'assemblage de lentille optique (30), comprenant un module de source lumineuse (31), un module de caméra (32) et un module d'interface (33) dans le canal de montage (13), le module de source lumineuse (31) et le module de caméra (32) étant installés dans le trou de serrage de montage (14), le module d'interface (33) étant installé sur le siège fixe de montage (15), avec l'extrémité de sortie s'étendant hors du siège fixe de montage (15), et le module de source lumineuse (31) et le module de caméra (32) étant connectés au module d'interface (33) par des lignes de données (34) ;
S300 : sceller des espaces formés après que la première coque (11) et la seconde coque (12) soient encliquetées par soudage à l'aide d'un dispositif de soudage ultrasonique, obtenant ainsi un produit primaire ;
S400 : insérer une structure de protection dans le trou de serrage de montage (14) du produit primaire, manchonner une structure de protection sur la partie s'étendant hors du siège fixe de montage (15) du module d'interface (33), et exécuter un moulage par injection secondaire sur la coque de revêtement (20) à l'extérieur du produit primaire pour obtenir un produit formé par moulage par injection secondaire, la poignée creuse (22) étant formée intégralement avec l'abaisse-langue (21) ;
S500 : détacher la structure de protection sur le produit issu du moulage par injection secondaire, et ébarber la coque de revêtement (20) pour obtenir le laryngoscope.

7. Procédé de traitement selon la revendication 6, dans lequel, à S100, des barres de positionnement (18) sont formées aux extrémités avec le trou de serrage de montage (14) de la première coque (11) et de la seconde coque (12) par moulage par injection, et après que la première coque (11) et la seconde coque (12) soient encliquetées pour former la lame (10), les barres de positionnement (18) sur la première coque (11) et la seconde coque (12) forment une partie de la structure du trou de serrage de montage (14).

8. Procédé de traitement selon la revendication 6, dans lequel, à S400, la coque de revêtement (20) issue du moulage par injection secondaire à l'extérieur du produit primaire comporte un abaisse-langue (21) et une poignée creuse (22), la structure de protection recouvrant la partie, s'étendant hors du siège fixe de montage (15) du module d'interface (33) forme un remplissage dans le processus de moulage par injection de la poignée (22), et la structure de production insérée dans le trou de serrage de montage (14) forme un remplissage d'un trou visible (23) dans la coque de revêtement (20).

9. Procédé de traitement selon la revendication 6, dans lequel, à S200, le module de source lumineuse (31), le module de caméra (32) et le module d'interface (33) sont recouverts de films d'isolation thermique et installés dans le canal de montage (13) avec les films d'isolation thermique.

10. Procédé de traitement selon la revendication 6, dans lequel, à S200, lorsque le module de source lumineuse (31) et le module de caméra (32) sont installés dans le trou de serrage de montage (14), une plaque de fenêtre transparente (17) est installée à l'extrémité extérieure du trou de serrage de montage (14).
